# EUROPEAN PATENT SPECIFICATION

(11) **EP 1 035 113 B1**
(45) Date of publication and mention of the grant of the patent: **06.10.2004**
(21) Application number: 00301810.8
(22) Date of filing: 06.03.2000
(51) Int. Cl.: C07D 207/34, C07C 257/02, C07C 233/69, C07C 233/66

(54) **Process for the preparation of 2-aryl-5-(perfluoroalkyl)pyrrole compounds from N-[1-chloro-1-(perfluoroalkyl)methyl]arylimidoyl chloride compounds**
Verfahren zur Herstellung von 2-Aryl-5-(perfluoroalkyl)pyrrolverbindungen aus N-[1-Chloro-1-(perfluoroalkyl)methyl]arylimidoylchlorid-Verbindungen
Procédé de préparation de composés 2-aryl-5-(perfluoroalkyl)pyrrole à partir de composés chlorure de N-[1-chloro-1-(perfluoroalkyl)methyl]arylimidoyle

(30) Priority: 09.03.1999 US 265339
(43) Date of publication of application: 13.09.2000
(73) Proprietor: BASF AKTIENGESELLSCHAFT, 67056 Ludwigshafen (DE)
(72) Inventor: Kameswaran, Venkataraman, Pennington, New Jersey 08534 (US)
(74) Representative: Pohl, Michael, Dr.

(56) References cited:
- EP-A- 0 531 702
- EP-A- 0 934 929
- US-A- 5 817 834
- ONYS'KO P P ET AL: "SIGMATROPIC ISOMERIZATIONS IN 2-AZA-ALLYLIC SYSTEMS. PART X. PROTOTROPIC AND CHLOROTROPIC REARRANGEMENTS IN FLUOROALKYL-SUBSTITUTED 1,3-DICHLORO-IMINES" JOURNAL OF FLUORINE CHEMISTRY,ELSEVIER SEQUOIA. LAUSANNE,CH, vol. 69, no. 3, 1994, pages 213-218, XP000944979 ISSN: 0022-1139
- TANAKA K ET AL: "N-ACYL-1-CHLORO-2,2,2-TRIFLUOROETHYLAMINE AS A 2,2,2-TRIFLUOROETHYLAMINE-BUILDING BLOCK" BULLETIN OF THE CHEMICAL SOCIETY OF JAPAN,JAPAN PUBLICATIONS TRADING CO. TOKYO,JP, vol. 66, no. 2, 1993, pages 661-663, XP000944717 ISSN: 0009-2673
- FARACI W S ET AL: "MECHANISM OF INACTIVATION OF ALANINE RACEMACE BY BETA,BETA,BETA-TRIFLUORALANINE" BIOCHEMISTRY,AMERICAN CHEMICAL SOCIETY. EASTON, PA,US, vol. 28, no. 2, 24 January 1989 (1989-01-24), pages 431-437, XP000944716 ISSN: 0006-2960
- MUSTAFA M E -S ET AL: "TRIFLUOROPYRUVIC ACID HYDRATE IN HETEROCYCLIC SYNTHESIS PART III: A NOVEL SYNTHESIS OF 4-(TRIFLUOROMETHYL)-OXAZOLONES AND OTHER RELATED COMPOUNDS" HETEROCYCLES,XX,XX, vol. 24, no. 6, 1986, pages 1541-1547, XP000944630 ISSN: 0385-5414

## Description

### BACKGROUND OF THE INVENTION

2-Aryl-5-(perfluoroalkyl)pyrrole compounds are useful as insecticidal and acaricidal agents. In addition, those compounds are also useful for the preparation of other insecticidal and acaricidal agents. In particular, 2-aryl-5-(perfluoroalkyl)pyrrole compounds are key intermediates in the preparation of arylpyrrole compounds such as chlorfenapyr. Accordingly, there is an ongoing search to discover new methods for the preparation of 2-aryl-5-(perfluoroalkyl)pyrrole compounds.

The prior art discloses that 2-aryl-5-(trifluoromethyl)pyrrole compounds may be prepared by reacting an N-(substituted benzyl)-2,2,2-trifluoro-acetimidoyl chloride compound with an α-halo-α,β-unsaturated nitrile, ester or nitro compound in the presence of a base. However, the prior art process is not entirely satisfactory because the required α-halo-α,β-unsaturated nitrile, ester or nitro compound is prepared in a two step -halogenation/dehydrohalogenation - process.

The prior art also discloses that 2-aryl-5-(trifluoromethyl)pyrrole compounds may be obtained in several steps from the appropriate aldehyde. These processes require the use of an aminonitrile intermediate which is obtained via the Strecker synthesis from the appropriate aldehyde. However, the use of the Strecker synthesis is not entirely satisfactory because of cyanide containing waste streams.

It is, therefore, an object of the present invention to provide a new process for the preparation of 2-aryl-5-(perfluoroalkyl)pyrrole compounds which avoids the use of α-halo-α,β-unsaturated nitrile, ester and nitro compounds and the Strecker synthesis.

It is also an object of this invention to provide a new process for the preparation of arylpyrrole compounds such as chlorfenapyr.

A further object of the present invention is to provide new intermediate compounds which are useful in the processes described hereinbelow.

Those and other objects of the present invention will become more apparent from the detailed description thereof set forth below.

### SUMMARY OF THE INVENTION

The present invention provides a new process for the preparation of 2-aryl-5-(perfluoroalkyl)pyrrole compounds having the structural formula I 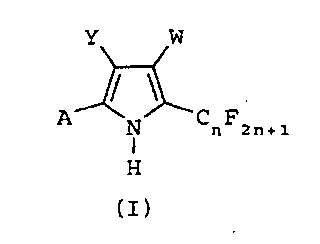 wherein
- W: is hydrogen or CₘF₂ₘ₊₁;
- Y: is CN, NO₂ or CO₂R;
- R: is C₁-C₄alkyl;
- m and n: are each independently an integer of 1, 2, 3, 4, 5, 6, 7 or 8;
- A: is 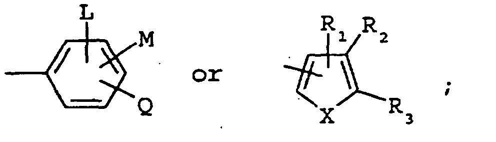
- L: is hydrogen or halogen;
- M and Q: are each independently hydrogen, halogen, CN, NO₂, C₁-C₄alkyl, C₁-C₄haloalkyl, C₁-C₄alkoxy, C₁-C₄haloalkoxy, C₁-C₄alkylthio, C₁-C₄haloalkylthio, C₁-C₄alkylsulfinyl, C₁-C₄haloalkylsulfinyl, C₁-C₄alkylsulfonyl, C₁-C₄haloalkylsulfonyl with the proviso than M cannot be hydrogen, or when M and Q are on adjacent positions they may be taken together with the carbon atoms to which they are attached to form a ring in which MQ represents the structure -OCH₂O-, -OCF₂O- or -CH=CH-CH=CH-;
- R₁, R₂ and R₃: are each independently hydrogen, halogen, NO₂, CHO or R₂ and R₃ may be taken together with the atoms to which they are attached to form a ring in which R₂R₃ is represented by the structure 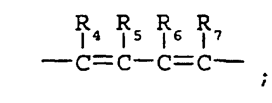
- R₄, R₅, R₆ and R₇: are each independently hydrogen, halogen, CN or NO₂; and
- X: is O or S,
which process comprises reacting an N-[1-chloro-1-(perfluoroalkyl)methyl]arylimidoyl chloride compound having the structural formula II 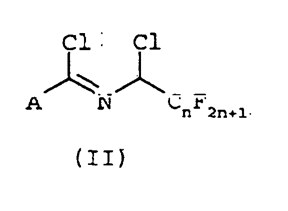 wherein A and n are as described above with a dieneophile compound having the structural formula III 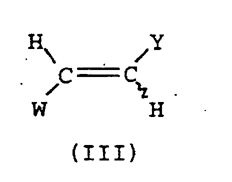 wherein W and Y are as described above and a base in the presence of a solvent.

The present invention further provides novel compounds having the structural formulas II, IV and V 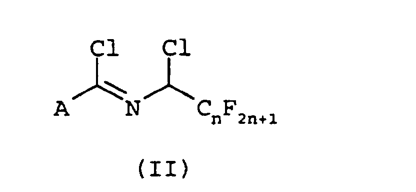 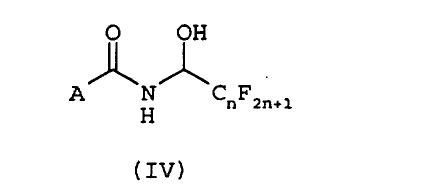 and 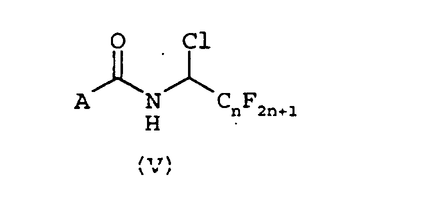 wherein n and A are as described hereinabove.

### DETAILED DESCRIPTION OF THE INVENTION

The process of the present invention preferably comprises reacting an N-[1-chloro-1-(perfluoroalkyl)methyl]arylimidoyl chloride compound of formula II with at least about one molar equivalent, preferably about one to four molar equivalents, of a dienophile compound of formula III and at least about one molar equivalent, preferably about one to four molar equivalents, of a base in the presence of a solvent preferably at a temperature range of about 5°C to 100°C to form 2-aryl-5-(perfluoroalkyl)pyrrole compounds of formula I.

. Alternatively, the formula I compounds may be prepared by forming the formula III dienophile compounds *in situ*. This process comprises reacting an N-[1-chloro-1-(perfluoroalkyl)methyl]arylimidoyl chloride compound of formula II with preferably about one to four molar equivalents of a substituted haloethane compound having the structural formula VI 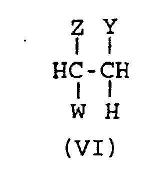 wherein W and Y are as described hereinabove and Z is Cl, Br or I, and at least about two molar equivalents, preferably about two to five molar equivalents, of a base in the presence of a solvent preferably at a temperature range of about 5°C to 100°C to form 2-aryl-5-(perfluoroalkyl)pyrrole compounds of formula I.

Advantageously, the present invention provides new processes for the preparation of 2-aryl-5-(perfluoroalkyl)pyrrole compounds which avoid the use of α-halo-α,β-unsaturated nitrile, ester and nitro compounds and the Strecker synthesis.

The formula I compounds of this invention may be isolated by conventional procedures such as dilution of the reaction mixture with water and filtration or, alternatively, extraction with a suitable solvent. Suitable extraction solvents include water-immiscible solvents such as ether, ethyl acetate, toluene, methylene chloride and the like.

Bases suitable for use in this invention include tri-(C₁-C₆alkyl)amines such as trimethylamine, triethylamine, tripropylamine, tributylamine, diisopropylethylamine and the like; alkali metal carbonates such as potassium carbonate and sodium carbonate; alkali metal hydroxides such as potassium hydroxide and sodium hydroxide; alkali metal acetates such as potassium acetate and sodium acetate; and heterocyclic tertiary amines including, but not limited to, 1,8-diazabicyclo[5.4.0]undec-7-ene (DBU); 1,5-diazabicyclo[4.3.0]non-5-ene (DBN); 1,4-diazabicyclo[2.2.2]octane; pyridine; substituted pyridines such as 2,6-dimethylpyridine, 2-methylpyridine, 3-methylpyridine, 4-methylpyridine and the like; quinoline; and substituted quinolines. Preferred bases include tri-(C₁-C₆alkyl) amines, 1,8-diazabicyclo[5.4.0]undec-7-ene, 1,5-diazabicyclo[4.3.0]non-5-ene, 1,4-diazabicyclo[2.2.2]octane, potassium carbonate and sodium carbonate.

Solvents suitable for use in the present invention include, but are not limited to, carboxylic acid amides such as N,N-dimethylformamide, N,N-dimethylacetamide and the like; N-substituted pyrrolidinones such as N-methylpyrrolidinone and the like; nitriles such as acetonitrile, propionitrile and the like; halogenated hydrocarbons such as methylene chloride, chloroform, carbon tetrachloride and the like; ethers such as tetrahydrofuran, dioxane and the like; sulfoxides such as dimethyl sulfoxide and the like; and mixtures thereof. Preferred solvents include carboxylic acid amides and nitriles and mixtures thereof. N,N-dimethylformamide and acetonitrile and mixtures thereof are especially preferred for use in the present invention.

Exemplary of halogen hereinabove are fluorine, chlorine, bromine and iodine. The terms "C₁-C₄haloalkyl", "C₁-C₄haloalkoxy", "C₁-C₄haloalkylthio", "C₁-C₄haloalkylsulfinyl" and "C₁-C₄haloalkylsulfonyl" are defined as a C₁-C₄alkyl, C₁-C₄alkoxy, C₁-C₄alkylthio, C₁-C₄alkylsulfinyl or C₁-C₄alkylsulfonyl group substituted with one or more halogen atoms, respectively.

When used herein the term alkyl, used as a group or part of a group, includes straight and branched alkyl groups, e.g. methyl, ethyl, n-propyl, i-propyl, n-butyl, i-butyl, s-butyl and t-butyl. When used herein the term haloalkyl, used as a group or part of a group, includes straight and branched haloalkyl groups, e.g. trifluoromethyl and trichloromethyl. Moiety A is preferably 4-chlorophenyl, 4-bromophenyl, 3,5-dichloro-phenyl, 3,4,5-trichlorophenyl or 4-trifluoromethylphenyl.

The present invention is especially useful for the preparation of formula I compounds wherein
- W: is hydrogen;
- Y: is CN;
- n: is 1 or 2;
- A: is 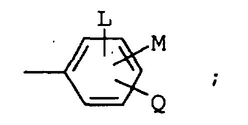
- L: is hydrogen or halogen; and
- M and Q: are each independently hydrogen, halogen, C₁-C₄haloalkyl or C₁-C₄haloalkoxy, with the proviso that M is not hydrogen.

In particular, the present invention is useful for the preparation of
2-(*p*-chlorophenyl)-5-(trifluoromethyl)pyrrole-3-carbonitrile;
2-(*p*-bromophenyl)-5-(trifluoromethyl)pyrrole-3-carbonitrile;
2-(3,5-dichlorophenyl)-5-(trifluoromethyl)pyrrole-3-carbonitrile;
2-(3,4,5-trichlorophenyl)-5-(trifluoromethyl)pyrrole-3-carbonitrile; and
2-[4-(trifluoromethyl)phenyl]-5-(trifluoromethyl)pyrrole-3-carbonitrile, among others.

The present invention also relates to N-[1-chloro-1-(perfluoroalkyl)methyl]arylimidoyl chloride compounds having the structural formula II 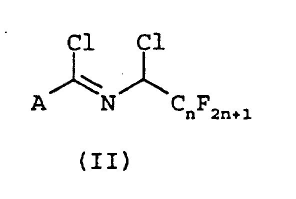 wherein
- n: is an integer of 1, 2, 3, 4, 5, 6, 7 or 8;
- A: is 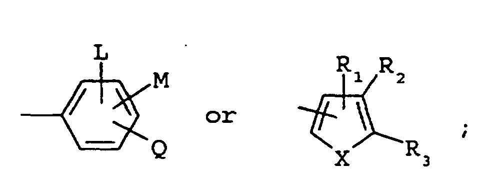
- L: is hydrogen or halogen;
- M and Q: are each independently hydrogen, halogen, CN, NO₂, C₁-C₄alkyl, C₁-C₄haloalkyl, C₁-C₄alkoxy, C₁-C₄haloalkoxy, C₁-C₄alkylthio, C₁-C₄haloalkylthio, C₁-C₄alkylsulfinyl, C₁-C₄haloalkylsulfinyl, C₁-C₄alkylsulfonyl, C₁-C₄haloalkylsulfonyl with the proviso that M cannot represent hydrogen or when M and Q are on adjacent positions they may be taken together with the carbon atoms to which they are attached to form a ring in which MQ represents the structure -OCH₂O-, -OCF₂O- or -CH=CH-CH=CH-;
- R₁, R₂ and R₃: are each independently hydrogen, halogen, NO₂, CHO or R₂ and R₃ may be taken together with the atoms to which they are attached to form a ring in which R₂R₃ is represented by the structure 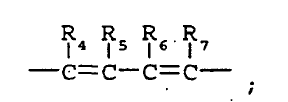
- R₄, R₅, R₆ and R₇: are each independently hydrogen, halogen, CN or NO₂; and
- X: is O or S.

Preferred formula II compounds of this invention are those wherein
- n: is 1 or 2;
- A: is 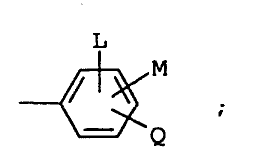
- L: is hydrogen or halogen; and
- M and Q: are each independently halogen, C₁-C₄haloalkyl or C₁-C₄haloalkoxy.

Formula II compounds which are particularly useful in the processes of this invention include
N-[1-chloro-(2,2,2-trifluoroethyl)]-4-chlorobenzimidoyl chloride;
N-[1-chloro-(2,2,2-trifluoroethyl)]-4-bromobenzimidoyl chloride;
N-[1-chloro-(2,2,2-trifluoroethyl)]-3,5-dichlorobenzimidoyl chloride;
N-[1-chloro-(2,2,2-trifluoroethyl)]-3,4,5-trichlorobenzimidoyl chloride; and
N-[1-chloro-(2,2,2-trifluoroethyl)]-4-(trifluoromethyl)benzimidoyl chloride, among others.

Starting N-[1-chloro-1-(perfluoroalkyl)methyl]arylimidoyl chloride compounds of formula II may be prepared, as shown in Flow Diagram I, by reacting an arylamide compound having the structural formula VII with a (perfluoroalkyl)aldehyde C₁-C₆alkyl hemiacetal compound having the structural formula VIII to form an N-[1-hydroxy-1-(perfluoroalkyl)methyl]arylamide compound having the structural formula IV, and reacting the formula IV compound with phosphorus pentachloride.

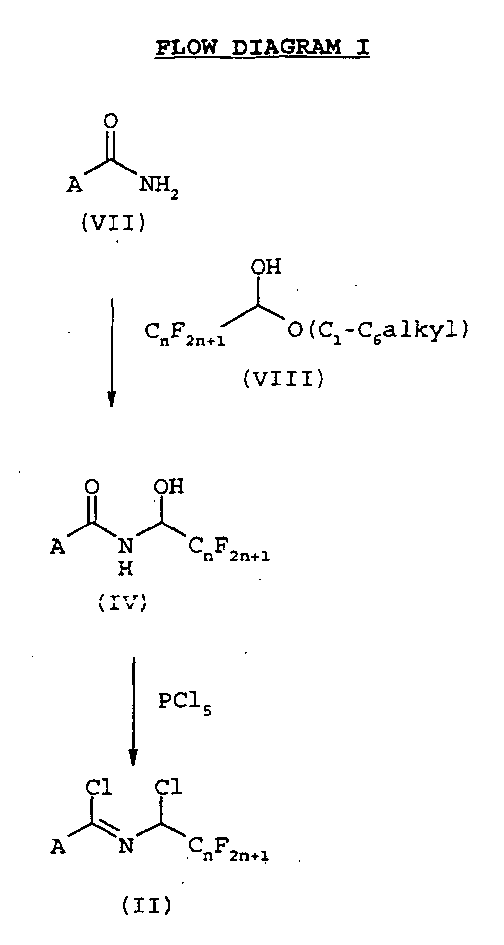

Alternatively, N-[1-chloro-1-(perfluoroalkyl)methyl]arylimidoyl chloride compounds of formula II may be prepared, as shown in Flow Diagram II, by reacting an N-[1-hydroxy-1-(perfluoroalkyl)methyl]arylamide of formula IV with phosphorus trichloride to form an N-[1-chloro-1-(perfluoroalkyl)methyl]arylamide compound having the structural formula V, and reacting the formula V compound with phosphorus pentachloride.

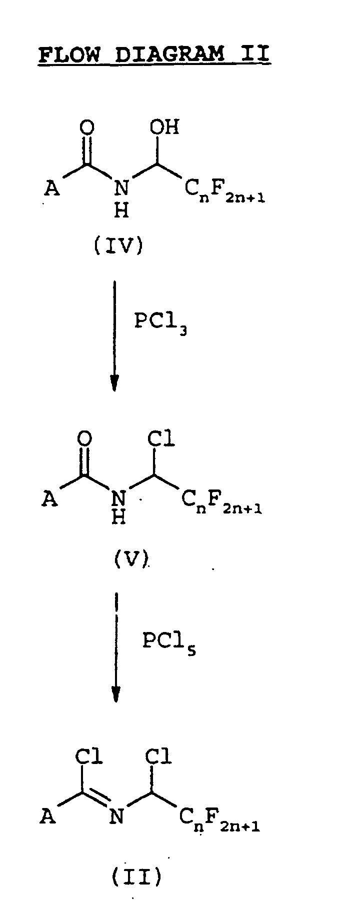

The present invention also relates to the formula IV and V compounds which are used to prepare the formula II compounds. In particular, the present invention provides N-[1-hydroxy-1-(perfluoroalkyl)methyl]arylamide compounds having the structural formula IV and N-[1-chloro-1-(perfluoroalkyl)methyl]arylamide compounds having the structural formula V 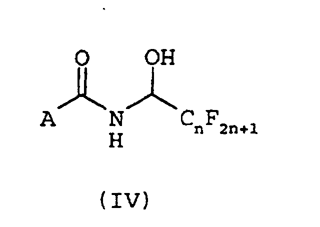 and 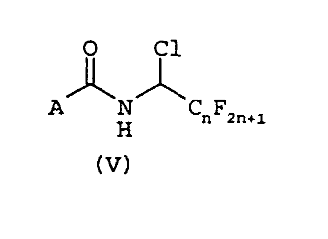 wherein
- n: is an integer of 1, 2, 3, 4, 5, 6, 7 or 8;
- A: is 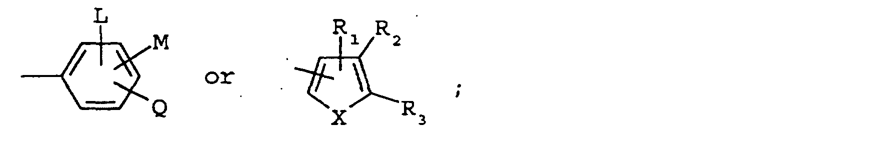
- L: is hydrogen or halogen;
- M and Q: are each independently hydrogen, halogen, CN, NO₂, C₁-C₄alkyl, C₁-C₄haloalkyl, C₁-C₄alkoxy, C₁-C₄haloalkoxy, C₁-C₄alkylthio, C₁-C₄haloalkylthio, C₁-C₄alkylsulfinyl, C₁-C₄haloalkylsulfinyl, C₁-C₄alkylsulfonyl, C₁-C₄haloalkylsulfonyl with the proviso that M cannot represent hydrogen or when M and Q are on adjacent positions they may be taken together with the carbon atoms to which they are attached to form a ring in which MQ represents the structure -OCH₂O-, -OCF₂O- or -CH=CH-CH=CH-;
- R₁, R₂ and R₃: are each independently hydrogen, halogen, NO₂, CHO or R₂ and R₃ may be taken together with the atoms to which they are attached to form a ring in which R₂R₃ is represented by the structure 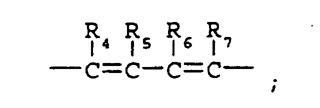
- R₄, R₅, R₆ and R₇: are each independently hydrogen, halogen, CN or NO₂; and
- X: is O or S.

Preferred formula IV and V compounds of this invention are those wherein
- n: is 1 or 2;
- A: is 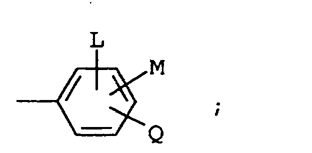
- L: is hydrogen or halogen; and
- M and Q: are each independently halogen, C₁-C₄haloalkyl or C₁-C₄haloalkoxy.

Formula IV compounds which are particularly useful for the preparation of arylpyrrole compounds include
N-(1-hydroxy-2,2,2-trifluoroethyl)-4-chlorobenzamide;
N-(1-hydroxy-2,2,2-trifluoroethyl)-4-bromobenzamide;
N-(1-hydroxy-2,2,2-trifluoroethyl)-3,5-dichlorobenzamide;
N-(1-hydroxy-2,2,2-trifluoroethyl)-3,4,5-trichlorobenzamide; and
N-(1-hydroxy-2,2,2-trifluoroethyl)-4-(trifluoromethyl)-benzamide, among others.

Formula V compounds which are particularly useful for the preparation of arylpyrrole compounds include
N-(1-chloro-2,2,2-trifluoroethyl)-4-chlorobenzamide;
N-(1-chloro-2,2,2-trifluoroethyl)-4-bromobenzamide;
N-(1-chloro-2,2,2-trifluoroethyl)-3,5-dichlorobenzamide;
N-(1-chloro-2,2,2-trifluoroethyl)-3,4,5-trichlorobenzamide; and
N-(1-chloro-2,2,2-trifluoroethyl)-4-(trifluoromethyl)-benzamide, among others.

The formula I compounds are useful for the control of insect and acarid pests. In addition, the formula I compounds may be used to prepare other arylpyrrole insecticidal and acaricidal agents having the structural formula IX 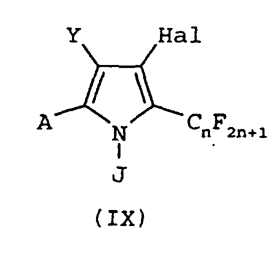 wherein
- Y: is CN, NO₂ or CO₂R;
- R: is C₁-C₄alkyl;
- n: is an integer of 1, 2, 3, 4, 5, 6, 7 or 8;
- A: is 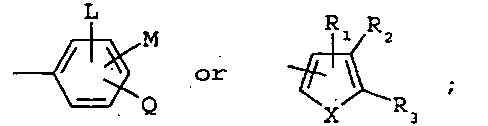
- L: is hydrogen or halogen;
- M and Q: are each independently hydrogen, halogen, CN, NO₂, C₁-C₄alkyl, C₁-C₄haloalkyl, C₁-C₄alkoxy, C₁-C₄haloalkoxy, C₁-C₄alkylthio, C₁-C₄haloalkylthio, C₁-C₄alkylsulfinyl, C₁-C₄haloalkylsulfinyl, C₁-C₄alkylsulfonyl, C₁-C₄haloalkylsulfonyl or when M and Q are on adjacent positions they may be taken together with the carbon atoms to which they are attached to form a ring in which MQ represents the structure -OCH₂O-, -OCF₂O- or -CH=CH-CH=CH-;
- R₁, R₂ and R₃: are each independently hydrogen, halogen, NO₂, CHO or R₂ and R₃ may be taken together with the atoms to which they are attached to form a ring in which R₂R₃ is represented by the structure 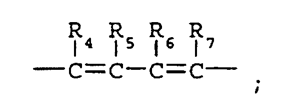
- R₄, R₅, R₆ and R₇: are each independently hydrogen, halogen, CN or NO₂;
- X: is O or S;
- Hal: is a halogen atom; and
- J: is hydrogen or C₁-C₆alkoxymethyl.

The present invention is especially useful for the preparation of arylpyrrole compounds of formula IX wherein
- Y: is CN;
- n: is 1 or 2;
- A: is 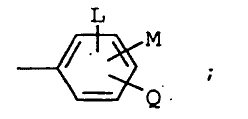
- L: is hydrogen or halogen;
- M and Q: are each independently hydrogen, halogen, C₁-C₄haloalkyl or C₁-C₄haloalkoxy, with the proviso that M is not hydrogen;
- Hal: is Br or Cl; and
- J: is hydrogen or ethoxymethyl.

In particular, the present invention is useful for the preparation of formula IX arylpyrrole compounds such as
4-bromo-2-(*p*-chlorophenyl)-1-(ethoxymethyl)-5-(trifluoromethyl)pyrrole-3-carbonitrile, (chlorfenapyr);
4-bromo-2-(3,5-dichlorophenyl)-1-(ethoxymethyl)-5-(trifluoromethyl)pyrrole-3-carbonitrile;
4-bromo-2-(3,5-dichlorophenyl)-5-(trifluoromethyl)pyrrole-3-carbonitrile; and
4-bromo-2-(*p*-chlorophenyl)-5-(trifluoromethyl)pyrrole-3-carbonitrile, among others.

Advantageously, formula IX arylpyrrole compounds may be prepared by a process which comprises:
(a) reacting an N-[1-chloro-1-(perfluoroalkyl)methyl]arylimidoyl chloride compound of formula II with a dienophile compound having the structural formula X 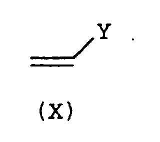 wherein Y is as described above and a base in the presence of a solvent to form a 2-aryl-5-(perfluoroalkyl)pyrrole compound having the structural formula XI 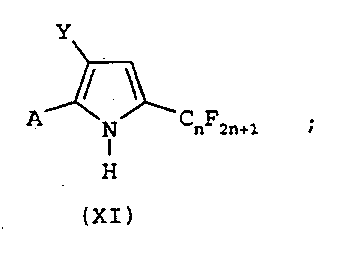
(b) halogenating the formula XI compound to form the arylpyrrole compound of formula IX wherein J is hydrogen; and
(c) optionally alkoxymethylating the formula IX compound wherein J is hydrogen to form the formula IX arylpyrrole compound wherein J is C₁-C₆alkoxymethyl.

Alternatively, arylpyrrole compounds of formula IX may be prepared by a process which comprises:
(a) reacting an N-[1-chloro-1-(perfluoroalkyl)methyl]arylimidoyl chloride compound of formula II with a substituted haloethane compound having the structural formula XII 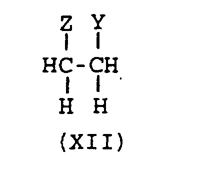 wherein Y is as described above and Z is Cl, Br or I, and at least about two molar equivalents of a base in the presence of a solvent to form a 2-aryl-5-(perfluoroalkyl)pyrrole compound having the structural formula XI 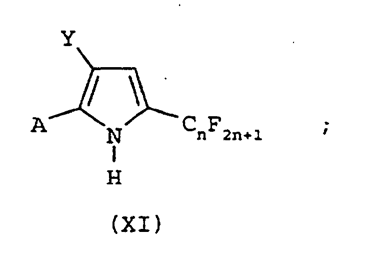
(b) halogenating the formula XI compound to form the arylpyrrole compound of formula IX wherein J is hydrogen; and
(c) optionally alkoxymethylating the formula IX compound wherein J is hydrogen to form the formula IX arylpyrrole compound wherein J is C₁-C₆alkoxymethyl.

Halogenation methods may be any known methods such as those described in U.S. Pat. Nos. 5,010,098 and 5,449,789.

Alkoxymethylation procedures suitable for use in this invention include conventional procedures known in the art (see, e.g., U.S. Pat. Nos. 5,010,098 and 5,359,090). In a preferred embodiment of this invention, the alkoxymethylation procedure comprises reacting a formula IX compound wherein J is hydrogen with a di-(C₁-C₆alkoxy)methane compound, N,N-dimethylformamide and phosphorous oxychloride in the presence of an aprotic solvent to form a reaction mixture and treating the reaction mixture with a tertiary amine.

In order to facilitate a further understanding of this invention, the following examples are presented primarily for the purpose of illustrating more specific details thereof. The scope of the invention should not be deemed limited by the examples, but encompasses the entire subject matter defined in the claims.

### EXAMPLE 1

### Preparation of N-(1-Hydroxy-2,2,2-trifluoroethyl)-4-chlorobenzamide

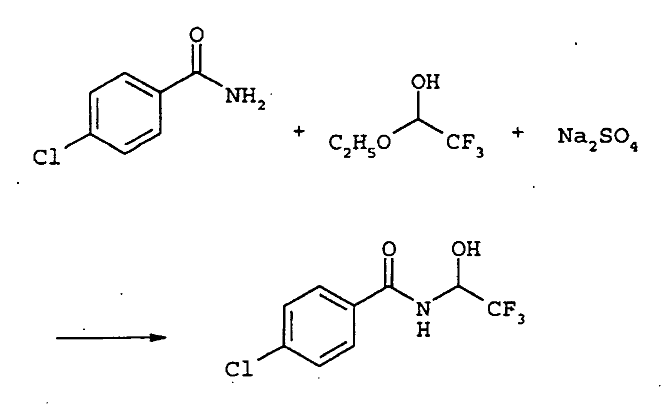

A solution of 4-chlorobenzamide (22.0 g, 0.141 mol) and trifluoroacetaldehyde ethyl hemiacetal (25.0 g as is, 22.5 g real, 0.156 mol) in dioxane (200 mL) is treated with anhydrous sodium sulfate (10 g, to dry the 10% water in the hemiacetal) and refluxed for 60 hours. The solids are filtered and the filtrate is evaporated to a solid. The solids are dissolved in about 200 mL of 15% ethyl acetate in heptane. Unreacted starting material (5.6 g) crystallizes out and is filtered. The title product is obtained from the mother liquors as a white crystalline solid (22.1 g, 82.9% based on recovery of starting material) : mp 139.5-140.5°C; characterized by ¹H and ¹⁹F NMR and Mass spectra. ¹H NMR (DMSO-d₆) δ 9.45 (d, J = 8.7 Hz, NH), 7.93, 7.54 ( AB with fine splitting, J = 8.4 Hz, ArH), 7.54 (broad s, OH), 5.90 (m, J = 8.7, 2.9, 5.8 Hz, CH); ¹⁹F NMR δ -80.3 (d, J = 5 Hz).

Following essentially the same procedure, but using the appropriately substituted benzamide, the following compounds are obtained:

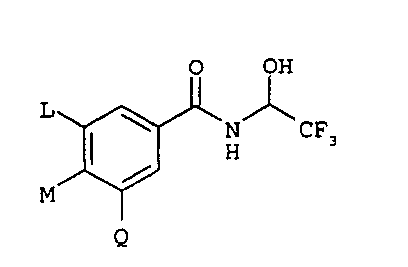

| **L** | **M** | **Q** | **mp°C** |
|---|---|---|---|
| Cl | H | Cl | 152.5-153 |
| H | Br | H | 148-148.5 |
| H | CF₃ | H | 124-124.5 |

### EXAMPLE 2

### Preparation of N-[1-Chloro-(2,2,2-trifluoroethyl)]-4-chlorobenzimidoyl chloride

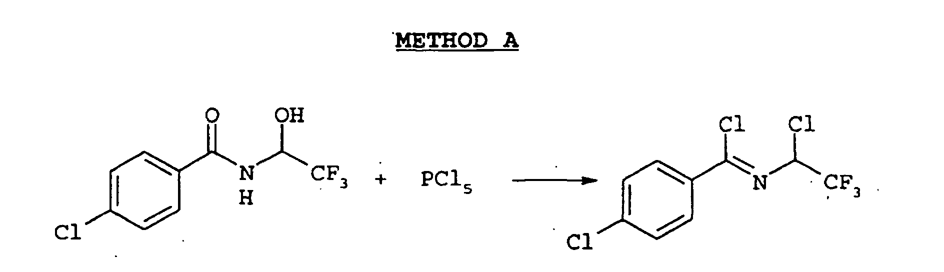

A mixture of N-(1-hydroxy-2,2,2-trifluoroethyl)-4-chlorobenzamide (22.1 g, 0.087 mol) in phosphorus oxychloride (8 mL) is treated with phosphorus pentachloride (40.0 g, 0.192 mol), heated to and held at 100°C for 15-20 minutes, cooled, and concentrated *in vacuo* to obtain a residue. The residue is distilled to give the title product as a clear liquid (22.2 g, 87.8% yield): bp 77-78°C (0.1 mm); characterized by IR, ¹H and ¹⁹F NMR, and Mass spectra. ¹H NMR (CDCl₃) δ 8.06, 7.44 ( d with fine splitting, J = 8.9 Hz, ArH), 5.92 (q, J = 4.9 Hz, CH); ¹⁹F NMR δ -77.9 (d, J = 5 Hz).

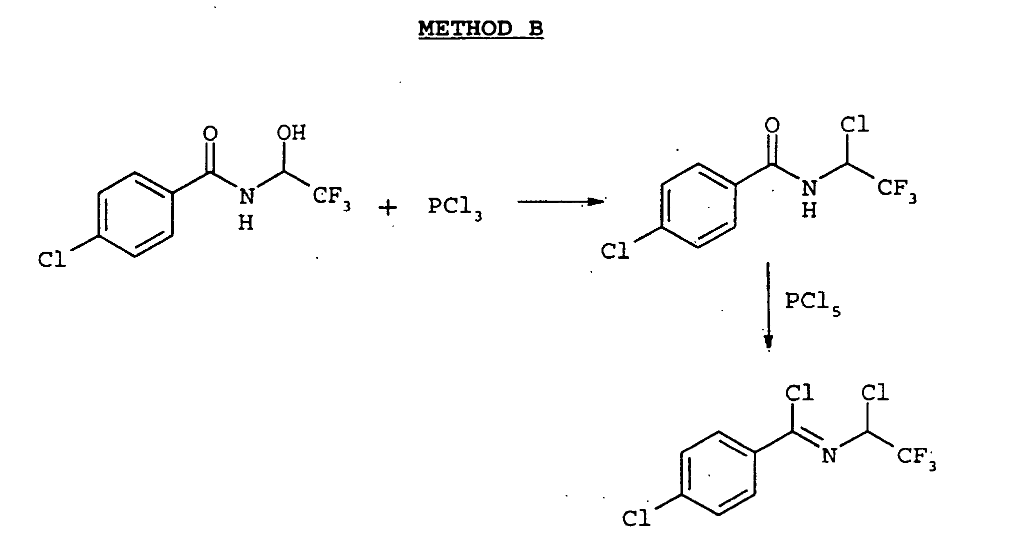

A mixture of N-(1-hydroxy-2,2,2-trifluoroethyl)-4-chlorobenzamide (16.3 g, 0.064 mol) in phosphorus oxychloride (10 mL) is treated with phosphorus trichloride (9.3 g, 0.675 mol) and heated to and held at 80°C for 15-20 minutes. ¹⁹F NMR shows clean and complete conversion to N-(1-chloro-2,2,2-trifluoroethyl)-4-chlorobenzamide. The reaction mixture is then cooled to room temperature, treated with phosphorus pentachloride (28.0 g, 0.135 mol), and heated to and held at 100°C for 1 hour. The phosphorus oxychloride is then removed *in vacuo* and the resultant residue is vacuum distilled to give the title product as a clear liquid (18.5 g, 100% yield): bp 94-96°C (0.5 mm).

Following essentially the same procedure as described in Method A, but using the appropriately substituted N-(1-hydroxy-2,2,2-trifluoroethyl)-4-benzamide, the following compounds are obtained:

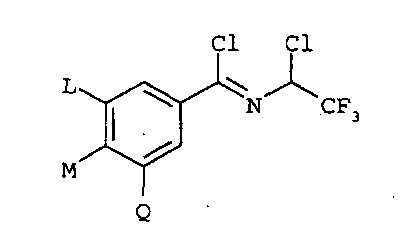

| **L** | **M** | **Q** | **bp** |
|---|---|---|---|
| Cl | H | Cl | 114°C (0.3mm) |
| H | Br | H | 95-96°C (0.07mm) |
| H | CF₃ | H | waxy solid |

### EXAMPLE 3

### Preparation of 2-(p-Chlorophenyl)-5-(trifluoromethyl)pyrrole-3-carbonitrile

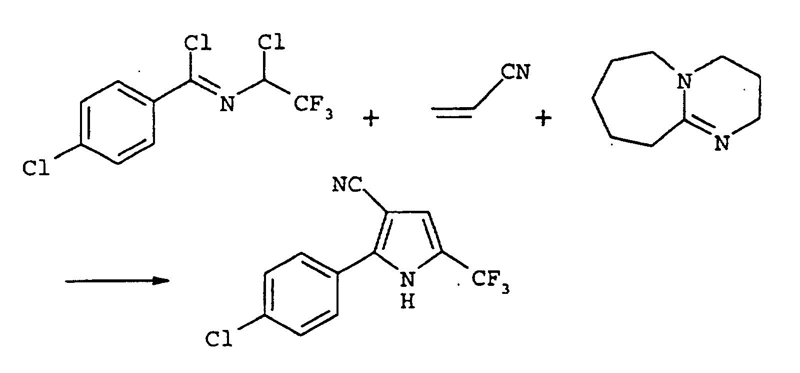

A solution of N-[1-chloro-(2,2,2-trifluoroethyl)]-4-chlorobenzimidoyl chloride (5.80 g, 0.02 mol) and acrylonitrile (1.33 g, 0.025 mol) in N,N-dimethylformamide (15 mL) is treated with 1,8-diazabicyclo[5.4.0]undec-7-ene (DBU, 8.53 g, 0.056 mol) over 1 hour while maintaining the temperature at 45°-50°C. The reaction mixture is then stirred at 50°C for 4 hours, quenched with dilute HCl, and extracted with ethyl acetate. The organic extract is concentrated *in vacuo* to obtain a residue. Flash chromatography of the residue on silica gel, packed and eluted with 20% ethyl acetate in heptane, and crystallization from heptane and small amount of ethyl acetate gives the title product as a white crystalline solid (2.1 g, 38.9% yield): mp 239-240°C (dec).

Following essentially the same procedure, but using the appropriately substituted N-[1-chloro-(2,2,2-trifluoroethyl)]benzimidoyl chloride, the following compounds are obtained:

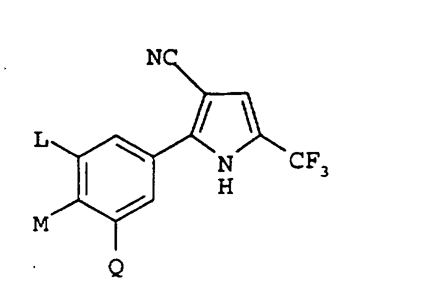

| **L** | **M** | **Q** | **mp°C** |
|---|---|---|---|
| Cl | H | Cl | 236.5-237 |
| H | Br | H | 248-249 |
| H | CF₃ | H | 216.5-218.5 |

### EXAMPLE 4

### Preparation of Methyl 2-(4-chlorophenyl)-5-(trifluoromethyl)pyrrole-3-carboxylate

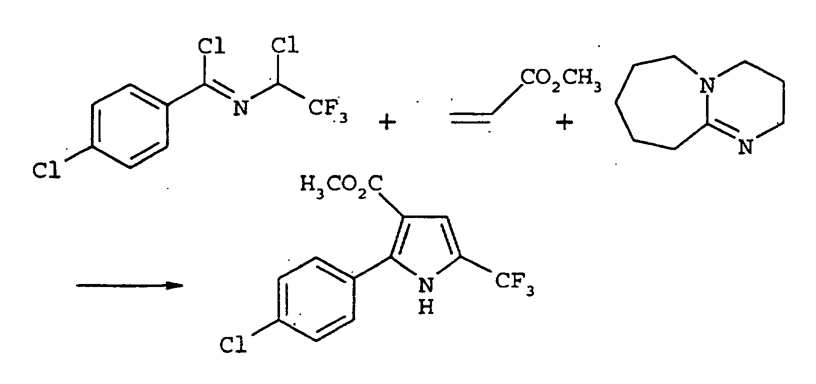

A solution of N-[1-chloro-(2,2,2-trifluoroethyl)]-4-chlorobenzimidoyl chloride (3.40 g, 0.012 mol) and methyl acrylate (1.26 g, 0.015 mol) in N,N-dimethylformamide (10 mL) is treated with 1,8-diazabicyclo[5.4.0]undec-7-ene (DBU, 5.0 g, 0.033 mol) over 1 hour. The reaction mixture is then held at 60°C for 15 minutes, quenched with dilute HCl, and extracted with ethyl acetate. The organic extract is concentrated *in vacuo* to obtain a residue. Flash chromatography of the residue on silica gel, packed and eluted with 20% ethyl acetate in heptane, and crystallization from heptane gives the title product as a yellow solid (0.95 g, 26.0% yield) which is identified by ¹H and ¹⁹F NMR spectral analyses.

### EXAMPLE 5

### Preparation of 4-Bromo-2-(4-chlorophenyl)-5-(trifluoromethyl)pyrrole-3-carbonitrile

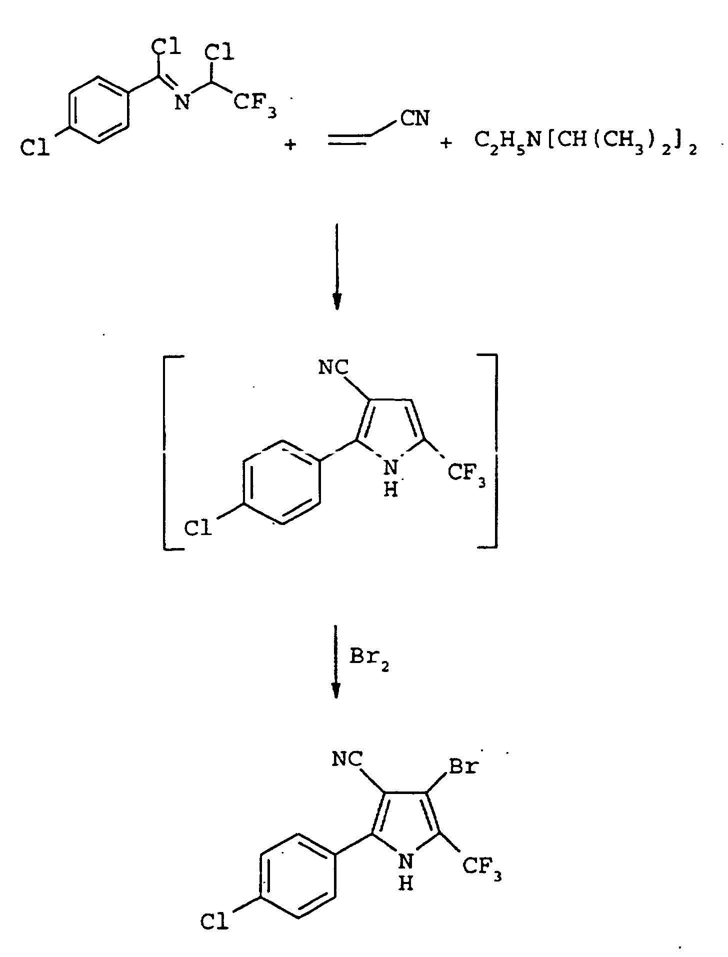

A solution of N-[1-chloro-(2,2,2-trifluoroethyl)]-4-chlorobenzimidoyl chloride (5.80 g, 0.02 mol) and acrylonitrile (1.33 g, 0.025 mol) in N,N-dimethylformamide (15 mL) under a nitrogen atmosphere is treated with N,N-diisopropylethylamine (DIPEA, 7.8 g, 0.06 mol) over 30 minutes, heated to and held at 45-47°C for 18 hours, cooled to room temperature, treated with bromine (3.2 g, 0.02 mol), stirred at room temperature for 1 hour, quenched with water, and extracted with ethyl acetate. The organic extract is concentrated *in vacuo* to obtain a residue. Flash column chromatography of the residue on silica gel, packed with 15% ethyl acetate in heptane and eluted with 20% ethyl acetate in heptane, gives the title product as white solid (1.6 g, 22.9% yield) which is identified by ¹H and ¹⁹F NMR spectral analyses.

### EXAMPLE 6

### Preparation of N-(1-Chloro-2,2,2-trifluoroethyl)-4-chlorobenzamide

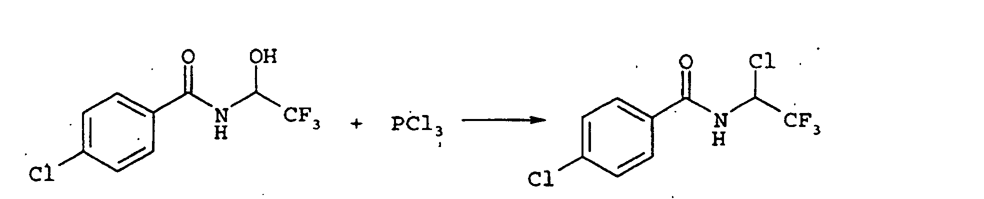

A mixture of N-(1-hydroxy-2,2,2-trifluoroethyl)-4-chlorobenzamide (2.53 g, 0.01 mol) in phosphorus oxychloride (2 mL) is treated with phosphorus trichloride (1.57 g, 0.012 mol), heated to and held at 80°C for 30 minutes, and concentrated *in vacuo* to obtain a residue. The residue is dissolved in hot heptane, decanted from the waxy phosphorus products, and crystallized to give the title product as a white crystalline solid (2.43 g, 89.3 % yield): mp 119.0-121.0°C; IR (Nujol) 3266, 1668 cm⁻¹; ¹H NMR (CDCl₃) δ 7.76 and 7.46 (AB with fine splitting, ArH), 6.86 (d, J = 8.5 Hz, NH, moves to 10.24 in DMSO-d₆), 6.55(m, CH) ; ¹⁹F NMR δ -77.7(d, J = 5 Hz).

Following essentially the same procedure, but using the appropriately substituted N-(1-hydroxy-2,2,2-trifluoroethyl)benzamide, the following compounds are obtained:

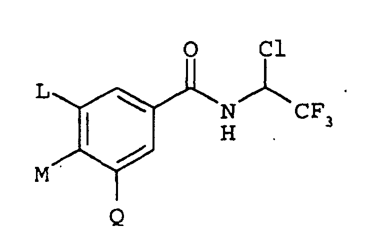

| **L** | **M** | **Q** | **mp°C** |
|---|---|---|---|
| Cl | H | Cl | 163.5-164 |
| H | Br | H | 135-136.5 |
| H | CF₃ | H | 122.5-123.5 |

## Claims

1. A compound having the formula II, IV or V 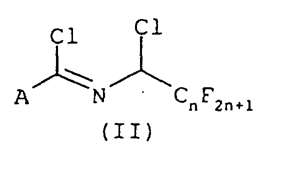 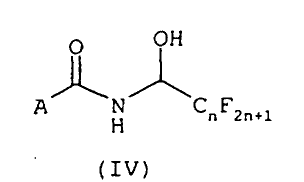 or 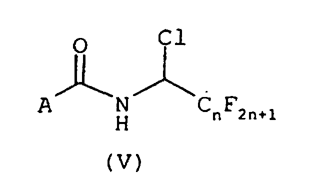 wherein
n is an integer of 1 to and including 8;
A is 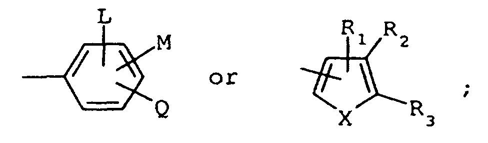
L is hydrogen or halogen;
M and Q are each independently hydrogen, halogen, CN, NO₂, C₁-C₄alkyl, C₁-C₄haloalkyl, C₁-C₄alkoxy, C₁-C₄haloalkoxy, C₁-C₄alkylthio, C₁-C₄haloalkylthio, C₁-C₄alkylsulfinyl, C₁-C₄haloalkylsulfinyl, C₁-C₄alkylsulfonyl or C₁-C₄haloalkylsulfonyl, with the proviso that M can not be hydrogen, or when M and Q are on adjacent positions they may be taken together with the carbon atoms to which they are attached to form a ring in which MQ represents the structure -OCH₂O-, -OCF₂O- or -CH=CH-CH=CH-;
R₁, R₂ and R₃ are each independently hydrogen, halogen, NO₂ or CHO, or R₂ and R₃ may be taken together with the atoms to which they are attached to form a ring in which R₂R₃ is represented by the structure 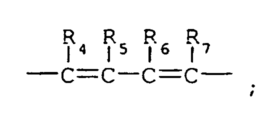
R₄, R₅, R₆ and R₇ are each independently hydrogen, halogen, CN or NO₂; and
X is O or S.

2. A compound as claimed in claim 1 wherein
n is 1 or 2;
A is 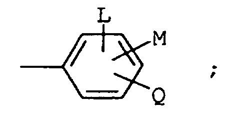
L is hydrogen or halogen; and
M and Q are each independently hydrogen, halogen, C₁-C₄haloalkyl or C₁-C₄haloalkoxy.

3. A compound as claimed in claim 1 or claim 2 which is selected from the group consisting of
N-[1-chloro-(2,2,2-trifluoroethyl)]-4-chlorobenzimidoyl chloride;
N-[1-chloro-(2,2,2-trifluoroethyl)]-4-bromobenzimidoyl chloride;
N-[1-chloro-(2,2,2-trifluoroethyl)]-3,5-dichlorobenzimidoyl chloride;
N-[1-chloro-(2,2,2-trifluoroethyl)]-3,4,5-trichlorobenzimidoyl chloride;
N-[1-chloro-(2,2,2-trifluoroethyl)]-4-(trifluoromethyl)benzimidoyl chloride;
N-(1-hydroxy-2,2,2-trifluoroethyl)-4-chlorobenzamide;
N-(1-hydroxy-2,2,2-trifluoroethyl)-4-bromobenzamide;
N-(1-hydroxy-2,2,2-trifluoroethyl)-3,5-dichlorobenzamide;
N- (1-hydroxy-2,2,2-trifluoroethyl)-3,4,5-trichlorobenzamide;
N-(1-hydroxy-2,2,2-trifluoroethyl)-4-(trifluoromethyl)benzamide.
N-(1-chloro-2,2,2-trifluoroethyl)-4-chlorobenzamide;
N-(1-chloro-2,2,2-trifluoroethyl)-4-bromobenzamide;
N-(1-chloro-2,2,2-trifluoroethyl)-3,5-dichlorobenzamide;
N-(1-chloro-2,2,2-trifluoroethyl)-3,4,5-trichlorobenzamide; and
N-(1-chloro-2,2,2-trifluoroethyl)-4-(trifluoromethyl)benzamide.

4. A process for the preparation of an N-[1-chloro-1-(perfluoroalkyl)methyl]arylimidoyl chloride compound of claim 1 having the formula II 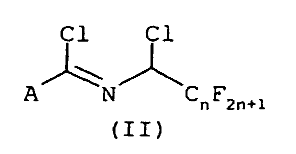 which process comprises reacting:
an N-[1-hydroxy-1-(perfluoroalkyl)methyl]arylamide compound having the formula IV 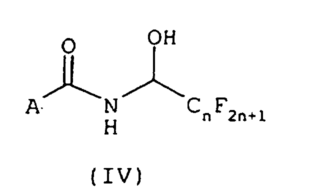 or
an N-[1-chloro-1-(perfluoroalkyl)methyl]arylamide compound having the formula V 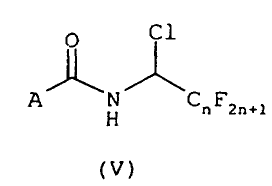
with phosphorous pentachloride, wherein n and A are as described in claim 1.

5. A process as claimed in claim 4 wherein
n is 1 or 2;
A is 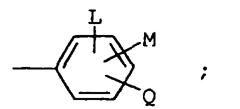
L is hydrogen or halogen; and
M and Q are each independently hydrogen, halogen, C₁-C₄haloalkyl or C₁-C₄haloalkoxy.

6. A process for the preparation of a 2-aryl-5-(perfluoroalkyl)pyrrole compound having the formula I 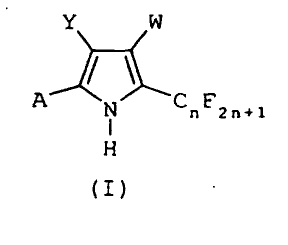 wherein
W is hydrogen or CₘF₂ₘ₊₁;
Y is CN, NO₂ or CO₂R;
R is C₁-C₄alkyl;
m and n are each independently an integer of 1 to and including 8; and
A is as described in claim 1,
which process comprises reacting an N-[1-chloro-1-(perfluoroalkyl)methyl]arylimidoyl chloride compound of claim 1 having the formula II 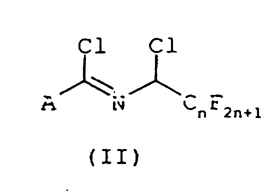 wherein A and n are as described above, with a dieneophile compound or a substituted haloethane compound, and a base, in the presence of a solvent, the dieneophile compound having the formula III and the substituted haloethane compound having the formula VI 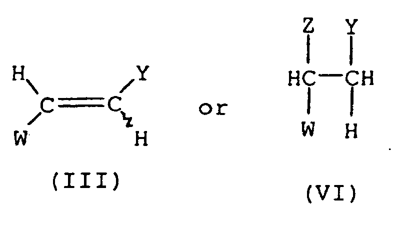 wherein W and Y are as described above, and Z is Cl, Br or I.

7. A process as claimed in claim 6 wherein
n is 1 or 2;
A is 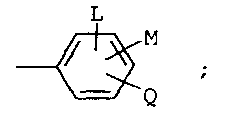
L is hydrogen or halogen; and
M and Q are each independently hydrogen, halogen, C₁-C₄haloalkyl or C₁-C₄haloalkoxy;
W is hydrogen; and
Y is CN.

8. A process as claimed in claim 6 or claim 7 wherein the base is selected from the group consisting of a tri-(C₁-C₆alkyl)amine, an alkali metal carbonate and a heterocyclic tertiary amine.

9. A process as claimed in any one of claims 6 to 8 wherein the solvent is selected from the group consisting of a carboxylic acid amide and a nitrile, and mixtures thereof.

10. A process as claimed in any one of claims 6 to 9 wherein the dieneophile is at about one to four molar equivalents and the base is at about one to four molar equivalents.

11. A process as claimed in any one of claims 6 to 10 wherein the substituted haloethane compound is at about one to four molar equivalents and the base is present in the amount of about two to five molar equivalents.

12. A process for the preparation of an arylpyrrole compound having the formula IX 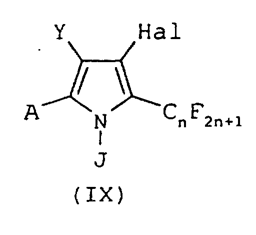 wherein
Y is CN, NO₂ or CO₂R;
R is C₁-C₄alkyl;
n and A are as described in claim 1;
Hal is a halogen atom; and
J is hydrogen or C₁-C₆alkoxymethyl,
which process comprises:
(a) reacting an N-[1-chloro-1-(perfluoroalkyl)methyl]arylimidoyl chloride compound of claim 1 having the formula II 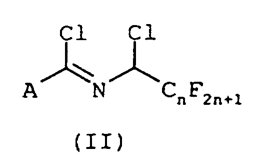 wherein A and n are as described above, with a dieneophile compound or a substituted haloethane compound, and a base, in the presence of a solvent, the dieneophile compound having the formula X and the substituted haloethane compound having the formula XII 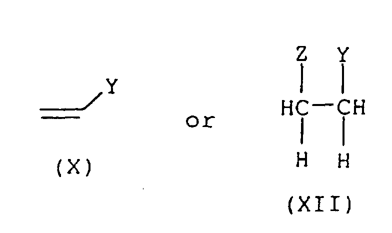 wherein Y is as described above, and Z is Cl, Br or I, to form a 2-aryl-5-(perfluoroalkyl)pyrrole compound having the formula XI 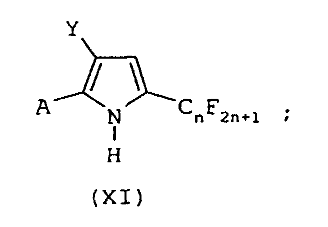
(b) halogenating the formula XI compound to form the arylpyrrole compound of formula IX wherein J is hydrogen; and
(c) optionally alkoxymethylating the formula IX compound.

13. A process as claimed in claim 12 wherein step (c) comprises reacting said formula IX compound with a di-(C₁-C₆alkoxy)methane compound, N,N-dimethylformamide and phosphorous oxychloride in the presence of an aprotic solvent to form a reaction mixture, and treating the reaction mixture with a tertiary amine.

## Patentansprüche

1. Verbindung der Formeln II, IV oder V 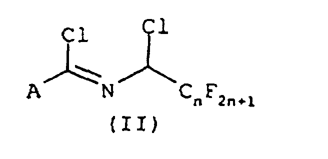 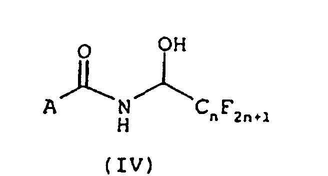 oder 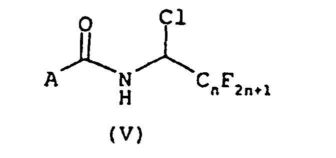 worin
n für eine ganze Zahl von 1 bis einschließlich 8 steht;
A für 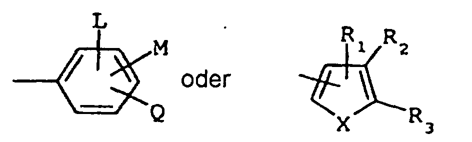 steht;
L für Wasserstoff oder Halogen steht;
M und Q jeweils unabhängig voneinander für Wasserstoff, Halogen, CN, NO₂, C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio, C₁-C₄-Alkylsulfinyl, C₁-C₄-Halogenalkylsulfinyl, C₁-C₄-Alkylsulfonyl oder C₁-C₄-Halogenalkylsulfonyl stehen, mit der Maßgabe, dass M nicht für Wasserstoff steht oder wenn M und Q, wenn sie einander benachbart sind, zusammen mit den Kohlenstoffatomen, an die sie gebunden sind, einen Ring bilden können, bei dem MQ für die Struktur -OCH₂O-, -OCF₂O- oder -CH=CH-CH=CH- steht;
R₁, R₂ und R₃ jeweils unabhängig voneinander für Wasserstoff, Halogen, NO₂ oder CHO stehen, oder R₂ und R₃ zusammen mit den Atomen, an die sie gebunden sind, einen Ring bilden können, worin R₂R₃ die Struktur 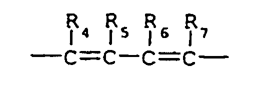 bedeutet;
R₄, R₅, R₆ und R₇ jeweils unabhängig voneinander für Wasserstoff, Halogen, CN oder NO₂ stehen; und
X für O oder S steht.

2. Verbindung nach Anspruch 1, worin
n für 1 oder 2 steht;
A für 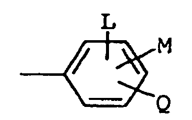 steht;
L für Wasserstoff oder Halogen steht; und
M und Q jeweils unabhängig voneinander für Wasserstoff, Halogen, C₁-C₄-Halogenalkyl oder C₁-C₄-Halogenalkoxy stehen.

3. Verbindung nach Anspruch 1 oder Anspruch 2, die ausgewählt ist unter:
N-[1-Chlor-(2,2,2-trifluorethyl)]-4-chlorbenzimidoylchlorid;
N-[1-Chlor-(2,2,2-trifluorethyl)]-4-brombenzimidoylchlorid;
N-[1-Chlor-(2,2,2-trifluorethyl)]-3,5-dichlorbenzimidoylchlorid;
N-[1-Chlor-(2,2,2-trifluorethyl)]-3,4,5-trichlorbenzimidoylchlorid;
N-[1-Chlor-(2,2,2-trifluorethyl)]-4-(trifluormethyl)benzimidoylchlorid;
N-(1-Hydroxy-2,2,2-trifluorethyl)-4-chlorbenzamid;
N-(1-Hydroxy-2,2,2-trifluorethyl)-4-brombenzamid;
N-(1-Hydroxy-2,2,2-trifluorethyl)-3,5-dichlorbenzamid;
N-(1-Hydroxy-2,2,2-trifluorethyl)-3,4,5-trichlorbenzamid;
N-(1-Hydroxy-2,2,2-trifluorethyl)-4-(trifluormethyl)benzamid;
N-(1-Chlor-2,2,2-trifluorethyl)-4-chlorbenzamid;
N-(1-Chlor-2,2,2-trifluorethyl)-4-brombenzamid;
N-(1-Chlor-2,2,2-trifluorethyl)-3,5-dichlorbenzamid;
N-(1-Chlor-2,2,2-trifluorethyl)-3,4,5-trichlorbenzamid; und
N-(1-Chlor-2,2,2-trifluorethyl)-4-(trifluormethyl)benzamid.

4. Verfahren zur Herstellung einer N-[1-Chlor-1-(perfluoralkyl)methyl]arylimidoylchlorid-Verbindung nach Anspruch 1 der Formel II, 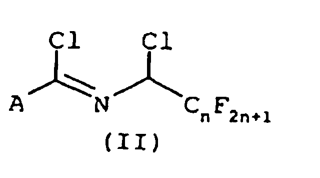 bei dem man
eine N-[1-Hydroxy-1-(perfluoralkyl)methyl]arylamid-Verbindung der Formel IV 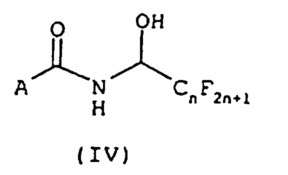 oder
eine N-[1-Chlor-1-(perfluoralkyl)methyl]arylamid-Verbindung der Formel V 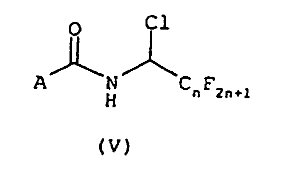 mit Phosphorpentachlorid umsetzt, wobei n und A wie in Anspruch 1 beschrieben sind.

5. Verfahren nach Anspruch 4, worin
n für 1 oder 2 steht;
A für 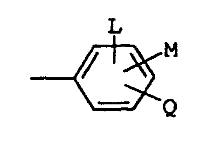 steht;
L für Wasserstoff oder Halogen steht; und
M und Q jeweils unabhängig voneinander für Wasserstoff, Halogen, C₁-C₄-Halogenalkyl oder C₁-C₄-Halogenalkoxy stehen.

6. Verfahren zur Herstellung einer 2-Aryl-5-(perfluoralkyl)pyrrol-Verbindung der Formel I 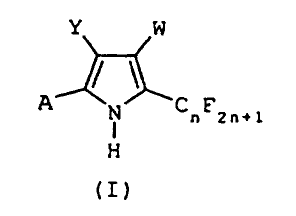 worin
W für Wasserstoff oder CₘF₂ₘ₊₁ steht;
Y für CN, NO₂ oder CO₂R steht;
R für C₁-C₄-Alkyl steht;
m und n jeweils unabhängig voneinander für eine ganze Zahl von 1 bis einschließlich 8 stehen; und
A wie in Anspruch 1 beschrieben ist,
bei dem man eine N-[1-Chlor-1-(perfluoralkyl)methyl]arylimidoylchlorid-Verbindung nach Anspruch 1 der Formel II 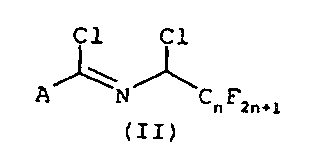 worin A und n wie oben beschrieben sind, mit einer dienophilen Verbindung oder einer substituierten Halogenethan-Verbindung und einer Base in Gegenwart eines Lösungsmittels umsetzt, wobei die dienophile Verbindung die Formel III und die substituierte Halogenethan-Verbindung die Formel VI aufweisen 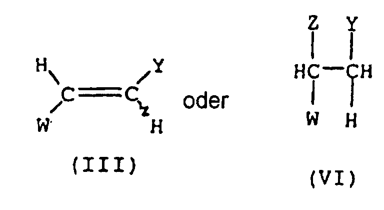 worin W und Y wie oben beschrieben sind, und Z für Cl, Br oder I steht.

7. Verfahren nach Anspruch 6, worin
n für 1 oder 2 steht;
A für 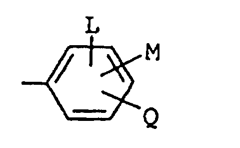 steht;
L für Wasserstoff oder Halogen steht; und
M und Q jeweils unabhängig voneinander für Wasserstoff, Halogen, C₁-C₄-Halogenalkyl oder C₁-C₄-Halogenalkoxy stehen;
W für Wasserstoff steht; und
Y für CN steht.

8. Verfahren nach Anspruch 6 oder 7, bei dem die Base ausgewählt ist unter einem Tri-( C₁-C₆-alkyl)amin, einem Alkalimetallcarbonat und einem heterocyclischen tertiären Amin.

9. Verfahren nach einem der Ansprüche 6 bis 8, bei dem das Lösungsmittel ausgewählt ist unter einem Carbonsäureamid und einem Nitril und Mischungen davon.

10. Verfahren nach einem der Ansprüche 6 bis 9, bei dem das Dienophil in einer Menge von etwa ein bis vier molaren Äquivalenten und die Base in einer Menge von etwa ein bis vier molaren Äquivalenten zugegen sind.

11. Verfahren nach einem der Ansprüche 6 bis 10, bei dem die substituierte Halogenethan-Verbindung in einer Menge von etwa ein bis vier molaren Äquivalenten und die Base in einer Menge von etwa zwei bis fünf molaren Äquivalenten zugegen sind.

12. Verfahren zur Herstellung einer Arylpyrrol-Verbindung der Formel IX 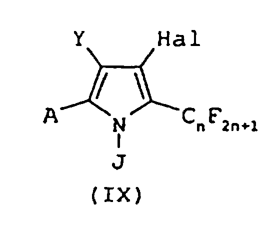 worin
Y für CN, NO₂ oder CO₂R steht;
R für C₁-C₄-Alkyl steht;
n und A wie in Anspruch 1 beschrieben sind;
Hal für ein Halogenatom steht; und
J für Wasserstoff oder C₁-C₆-Alkoxymethyl steht;
bei dem man
(a) eine N-[1-Chlor-1-(perfluoralkyl)methyl]arylimidoylchlorid-Verbindung gemäß Anspruch 1 der Formel II, 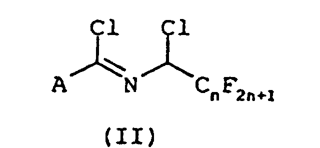 worin A und n wie oben beschrieben sind, mit einer dienophilen Verbindung oder einer substituierten Halogenethan-Verbindung und einer Base in Gegenwart eines Lösungsmittels umsetzt, wobei die dienophile Verbindung die Formel X und die substituierte Halogenethan-Verbindung die Formel XII aufweisen 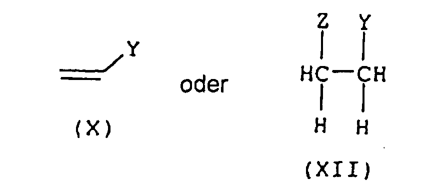 worin Y wie oben beschrieben ist, und Z für Cl, Br oder I steht, wobei eine 2-Aryl-5-(perfluoralkyl)pyrrol-Verbindung der Formel XI 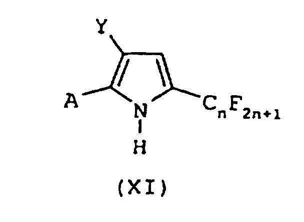 gebildet wird;
(b) die Verbindung der Formel XI halogeniert, wobei die Arylpyrrol-Verbindung der Formel IX gebildet wird, worin J für Wasserstoff steht; und
(c) gegebenenfalls die Verbindung der Formel IX alkoxymethyliert.

13. Verfahren nach Anspruch 12, wobei Schritt c) das Umsetzen der Verbindung der Formel IX mit einer Di-(C₁-C₆-alkoxy)methan-Verbindung, N,N-Dimethylformamid und Phosphoroxychlorid in Gegenwart eines aprotischen Lösungsmittels unter Bildung eines Reaktionsgemisches und Behandeln des Reaktionsgemisches mit einem tertiären Amin umfasst.

## Revendications

1. Composé présentant la formule II, IV ou V 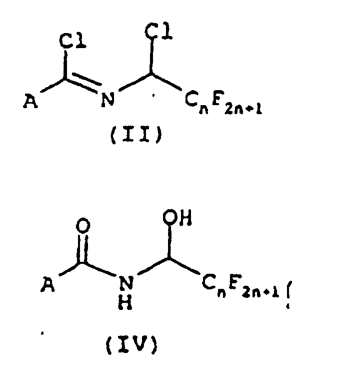 ou 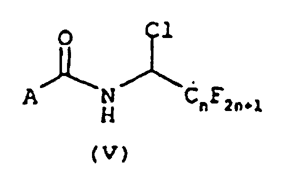 dans laquelle
n est un entier de 1 à 8 compris ;
A représente 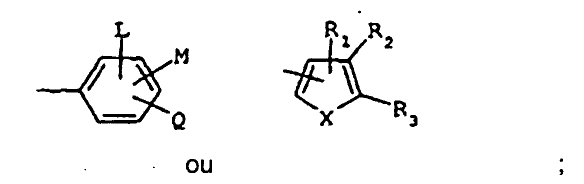
L représente hydrogène ou halogène ;
M et Q représentent, chacun indépendamment, hydrogène, halogène, CN, NO₂, alkyle en C₁ à C₄, halogénoalkyle en C₁ à C₄, alcoxy en C₁ à C₄, halogénoalcoxy en C₁ à C₄, (alkyle en C₁ à C₄)thio, halogéno(alkyle en C₁ à C₄)thio, (alkyle en C₁ à C₄)sulfinyle, halogéno(alkyle en C₁ à C₄)sulfinyle, (alkyle en C₁ à C₄)sulfonyle ou halogéno(alkyle en C₁ à C₄)sulfonyle, à condition que M ne puisse pas être hydrogène, ou lorsque M et Q se trouvent sur des positions adjacentes, ils peuvent être pris ensemble avec les atomes de carbone auxquels ils sont attachés pour former un cycle dans lequel MQ représente la structure -OCH₂O-, -OCF₂O- ou -CH=CH-CH=CH- ;
R₁, R₂ et R₃ représentent, chacun indépendamment, hydrogène, halogène, NO₂, ou CHO, ou R₂ et R₃ peuvent être pris ensemble avec les atomes auxquels ils sont attachés pour former un cycle dans lequel R₂R₃ est représenté par la structure 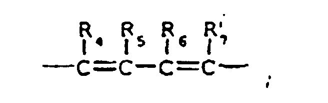
R₄, R₅, R₆ et R₇ représentent, chacun indépendamment, hydrogène, halogène, CN ou NO_{2;} et
X représente O ou S.

2. Composé selon la revendication 1, dans lequel
n représente 1 ou 2 ;
A représente 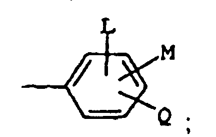
L représente hydrogène ou halogène ; et
M et Q représentent, chacun indépendamment, hydrogène, halogène, halogénoalkyle en C₁ à C₄ ou halogénoalcoxy en C₁ à C₄.

3. Composé selon la revendication 1 ou 2, qui est choisi dans le groupe constitué par
le chlorure de N- [1-chloro-(2,2, 2-trifluoroéthyl)]-4-chlorobenzimidoyle ;
le chlorure de N-[1-chloro-(2,2,2-trifluoroéthyl)]-4-bromobenzimidoyle ;
le chlorure de N-[1-chloro-(2,2,2-trifluoroéthyl)]-3,5-dichlorobenzimidoyle ;
le chlorure de N-[1-chloro-(2,2,2-trifluoroéthyl)]-3,4,5-trichlorobenzimidoyle ;
le chlorure de N-[1-chloro-(2,2,2-trifluoroéthyl)]-4-(trifluorométhyl)benzimidoyle ;
le N-(1-hydroxy-2,2,2-trifluoroéthyl)-4-chlorobenzamide;
le N-(1-hydroxy-2,2,2-trifluoroéthyl)-4-bromobenzamide;
le N- (1-hydroxy-2,2,2-trifluoroéthyl)-3,5-dichlorobenzamide;
le N-(1-hydroxy-2,2,2-trifluoroéthyl)-3,4,5-trichlorobenzamide;
le N-(1-hydroxy-2,2,2-trifluoroéthyl)-4-(trifluorométhyl)benzamide ;
le N-(1-chloro-2,2,2-trifluoroéthyl)-4-chlorobenzamide;
le N-(1-chloro-2,2,2-trifluoroéthyl)-4-bromobenzamide;
le N-(1-chloro-2,2,2-trifluoroéthyl)-3,5-dichlorobenzamide;
le N-(1-chloro-2,2,2-trifluoroéthyl)-3,4,5-trichlorobenzamide; et
le N-(1-chloro-2,2,2-trifluoroéthyl)-4-(trifluorométhyl)benzamide.

4. Procédé pour la préparation d'un composé chlorure de N-[1-chloro-1-(perfluoroalkyl)méthyl]arylimidoyle selon la revendication 1 présentant la formule II 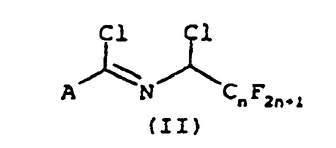 le procédé comprenant la réaction :
d'un composé N-[1-hydroxy-1-(perfluoroalkyl)méthyl]arylamide présentant la formule IV 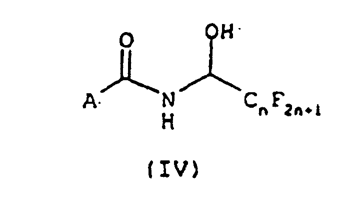 ou
d'un composé N-[1-chloro-1-(perfluoroalkyl)méthyl]arylamide présentant la formule V 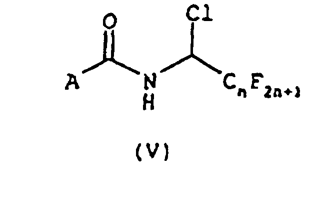 avec du pentachlorure phosphoreux, où n et A sont tels que décrits dans la revendication 1.

5. Procédé selon la revendication 4, dans lequel
n représente 1 ou 2 ;
A représente 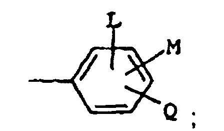
L représente hydrogène ou halogène ; et
M et Q représentent, chacun indépendamment, hydrogène, halogène, halogénoalkyle en C₁ à C₄ ou halogénoalcoxy en C₁ à C₄.

6. Procédé pour la préparation d'un composé 2-aryl-5-(perfluoroalkyl)pyrrole présentant la formule I 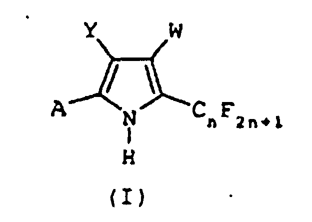 dans laquelle
W représente hydrogène ou CₘF₂ₘ₊₁ ;
Y représente CN, NO₂ ou CO₂R;
R représente alkyle en C₁ à C₄ ;
m et n représentent chacun indépendamment un entier de 1 à 8 compris ; et
A est tel que décrit dans la revendication 1,
le procédé comprenant la réaction d'un composé chlorure de N-[1-chloro-1-(perfluoroalkyl)méthyl]arylimidoyle selon la revendication 1, présentant la formule II 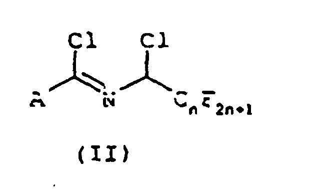 dans laquelle A et n sont tels que décrits ci-dessus, avec un composé diénophile ou un composé halogénoéthane substitué et une base en présence d'un solvant, le composé diénophile présentant la formule III et le composé halogénoéthane substitué présentant la formule VI 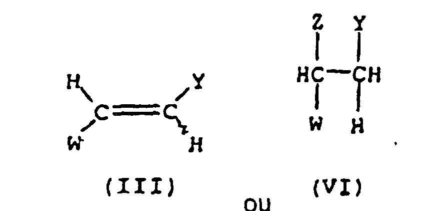 dans lesquelles W et Y sont tels que décrits ci-dessus et Z représente Cl, Br ou I.

7. Procédé selon la revendication 6, dans lequel
n représente 1 ou 2 ;
A représente 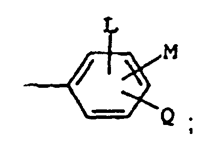
L représente hydrogène ou halogène ; et
M et Q représentent, chacun indépendamment, hydrogène, halogène, halogénoalkyle en C₁ à C₄ ou halogénoalcoxy en C₁ à C₄ ;
W représente hydrogène ;et
Y représente CN.

8. Procédé selon la revendication 6 ou 7, dans lequel la base est choisie dans le groupe constitué par une tri-(alkyle en C₁ à C₆)amine, un carbonate de métal alcalin et une amine tertiaire hétérocyclique.

9. Procédé selon l'une quelconque des revendications 6 à 8, dans lequel le solvant est choisi dans le groupe constitué par un amide de l'acide carboxylique et un nitrile et les mélanges de ceux-ci.

10. Procédé selon l'une quelconque des revendications 6 à 9, dans lequel le diénophile est présent en environ un à quatre équivalents molaires et la base est présente en environ un à quatre équivalents molaires.

11. Procédé selon l'une quelconque des revendications 6 à 10, dans lequel le composé halogénoéthane substitué est présent en environ un à quatre équivalents molaires et la base est présente en une quantité d'environ deux à cinq équivalents molaires.

12. Procédé pour la préparation d'un composé arylpyrrole présentant la formule IX 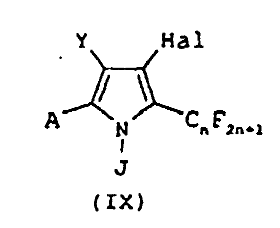 dans laquelle
Y représente CN, NO₂, ou CO₂R;
R représente alkyle en C₁ à C₄ ;
n et A sont tels que décrits dans la revendication 1 ;
Hal représente un atome d'halogène ; et
J représente hydrogène ou (alcoxy en C₁ à C₆)méthyle,
le procédé comprenant
(a) la réaction d'un composé chlorure de N-[1-chloro-1-(perfluoroalkyl)méthyl]arylimidoyle selon la revendication 1, présentant la formule II 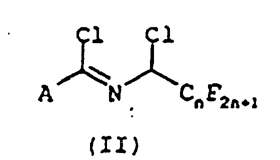 dans laquelle A et n sont tels que décrits ci-dessus, avec un composé diénophile ou un composé halogénoéthane substitué, et une base en présence d'un solvant, le composé diénophile présentant la formule X et le composé halogénoéthane substitué présentant la formule XII 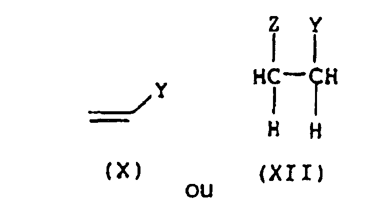 dans lesquelles Y est tel que décrit ci-dessus et Z représente Cl, Br ou I, pour former un composé 2-aryl-5-(perfluoroalkyl)pyrrole présentant la formule XI, 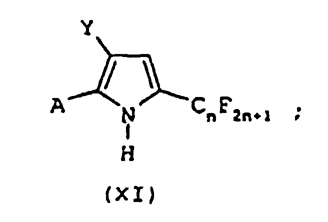
(b) l'halogénation du composé de formule XI pour former le composé arylpyrrole de formule IX dans laquelle J représente hydrogène et
(c) éventuellement l'alcoxyméthylation du composé de formule IX.

13. Procédé selon la revendication 12, dans lequel l'étape (c) comprend la réaction dudit composé de formule IX avec un composé di(alcoxy en C₁ à C₆)méthane, du N,N-diméthylformamide et de l'oxychlorure phosphoreux en présence d'un solvant aprotique pour former un mélange réactionnel et le traitement du mélange réactionnel avec une amine tertiaire.
